## Europäisches Patentamt

## European Patent Office

## Office européen des brevets

(19)

(11) Veröffentlichungsnummer: **0 246 469**
**B1**

(12) **EUROPÄISCHE PATENTSCHRIFT**

(45) Veröffentlichungstag der Patentschrift:
16.08.90

(51) Int. Cl.⁵: **B01J 13/02, A61K 9/50**

(21) Anmeldenummer: **87106203.0**

(22) Anmeldetag: **29.04.87**

(54) Verfahren zur Verkapselung von biologisch aktivem Material.

(30) Priorität: **03.05.86 DE 3615043**

(43) Veröffentlichungstag der Anmeldung:
**25.11.87 Patentblatt 87/48**

(45) Bekanntmachung des Hinweises auf die Patenterteilung:
**16.08.90 Patentblatt 90/33**

(84) Benannte Vertragsstaaten:
**AT BE CH DE ES FR GB GR IT LI LU NL SE**

(56) Entgegenhaltungen:
**WO-A-86/03676**
**DE-A- 3 012 233**
**DE-A- 3 209 127**
**FR-A- 2 194 484**
**FR-A- 2 275 250**
**US-A- 3 567 650**

(73) Patentinhaber: **HOECHST AKTIENGESELLSCHAFT,**
**Postfach 80 03 20, D-6230 Frankfurt am Main 80(DE)**

(72) Erfinder: **Bader, Hubert, Dr., Im Münchfeld 23,**
**D-6500 Mainz(DE)**
Erfinder: **Rüppel, Diether, Dr., Karl-König-Weg 1,**
**D-6230 Frankfurt am Main 80(DE)**
Erfinder: **Walch, Axel, Dr., Hans-Sachs-Strasse 5,**
**D-6000 Frankfurt am Main(DE)**

**Beschreibung**

Die Immobilisierung von Enzymen bzw. lebendem Zellmaterial ist weithin bekannt. Durch den Einschluß von aktivem Zellmaterial in Mikrokapseln, wie es z.B. in "Artificial Cells" T.M.S. Chang und C.C. Thomas Publ., Springfield Illinois (1972) beschrieben ist, läßt sich durch die Kompartimentierung unter Beibehaltung einer möglichst großen Oberfläche die biologische Aktivität des Materials erhalten bzw. verbessern.

In der deutschen Offenlegungsschrift 30 12 233 (US 4 352 883, US 4 391 909, US 4 407 957, US 4 495 288) wird ein Verfahren zum Einkapseln von Gewebe oder Einzelzellen beschrieben. Das Zellmaterial soll lebensfähig in geschütztem Zustand in einer Membran eingeschlossen sein, die zur Erhaltung der normalen Stoffwechselfunktionen der Zellen für Nährstoffe, Ionen, Sauerstoff und andere niedermolekulare Stoffe durchlässig ist. Das einzukapselnde Material wird in einem Medium suspendiert, das einen wasserlöslichen, in Tröpfchen gelierbaren Stoff enthält, um für das Gewebe eine vorübergehende schützende Umhüllung zu schaffen. Aufgrund der Empfindlichkeit des Zellmaterials können für die reversible Gelierung über ein Elektrolytmilieu nur sehr spezielle Puffermedien eingesetzt werden. Bevorzugte Stoffe für die Bildung der temporären Kapseln sind natürliche Polysaccharid-Gummiharze, die a) bei einer Änderung der Bedingungen, etwa des pH-Werts oder bei Zugabe von mehrwertigen Kationen, wie Ca$^{2+}$, zur reversiblen Bildung einer formhaltigen Masse befähigt sind und die b) mit Polybasen, deren Amingruppen mit sauren Polysaccharid-Bestandteilen reagieren können, dauerhaft komplexierbar sind. Nach Bildung der dauerhaften semipermeablen Membran kann die temporäre Kapsel durch Herstellung der Bedingungen, unter denen der Stoff flüssig ist, aufgelöst werden. Dies ist insbesondere erforderlich, wenn es sich um äußerst empfindliches, biologisch aktives Material handelt, das durch die zahlreichen, ionischen Gruppen des Polysaccharid-Harzes im Zellwachstum bzw. im Stoffwechsel behindert wird.

In der Offenlegungsschrift DE 32 09 127 (US 4 409 331) wird ein Verfahren beschrieben, in dem Zellen nach der obengenannten Methode eingekapselt werden, um Stoffwechselprodukte zu gewinnen.

Biologisch aktives Material kann auch in Proteine mit weitgehend neutraler Nettoladung eingeschlossen werden, wie in der Europäischen Anmeldung 0 129 619 erwähnt ist. Dabei treten jedoch Probleme auf, die z.B. durch leichtere mikrobiologische Kontamination oder geringere Reproduzierbarkeit der Proteine hervorgerufen werden, aber vor allem durch die Tatsache, daß ein reversibler Gel-/Sol-Übergang nicht möglich ist.

Nach der FR-A 2 194 434 hat man auch schon Enzym- oder Mikroorganismen-Suspensionen in polymere Fäden, beispielsweise aus Celluloseacetat, Cellulosephthalat, Hydroxypropylmethylcellulosephthalat oder Nitrocellulose, eingeschlossen.

Bei Verwendung von Agarose, Stärke oder Johannisbrotkernmehl, als weitgehend neutrale Gelbildner, für die temporäre Kapsel tritt die Verflüssigung der Gele erst durch Erhitzen auf vielfach unphysiologische Temperaturen von über 50°C ein.

Alle bisher für die Ausbildung der temporären Kapsel eingesetzten Gele zeigen eine Verflüssigung nur bei Erhöhung der Temperaturen, teilweise, wie schon erwähnt, auch nur bei Erhitzen auf im allgemeinen unphysiologische Temperaturen. Es werden bislang keine Gelbildner mit "umgekehrtem" Temperaturverhalten für einfache Verfahren zur reversiblen Verkapselung von biologisch aktivem Material verwendet. Als "umgekehrtes" Temperaturverhalten wird die Verflüssigung der Gele bei einer niedrigeren Temperatur als der Geliertemperatur bezeichnet.

Überraschend wurde nun gefunden, daß Gele mit umgekehrtem Temperaturverhalten sehr gut zur Verkapselung von biologisch aktivem Material eingesetzt werden können. Insbesondere die Verwendung nicht-ionischer Gele hat sich als vorteilhaft erwiesen, da elektrostatische Wechselwirkungen mit den Zellen vermieden werden. Die erfindungsgemäß hergestellten Kapseln können in den üblichen wäßrigen Kulturmedien suspendiert werden, wobei die Aktivität des eingeschlossenen Materials erhalten bleibt und Stoffwechselprodukte synthetisiert werden.

Die Erfindung betrifft somit:

1. Ein Verfahren zum Einkapseln von biologisch aktivem Material, das dadurch gekennzeichnet ist, daß zur Ausbildung der Kapseln ein Polysaccharid mit umgekehrtem thermoreversiblen Temperaturverhalten eingesetzt wird, wobei diese Eigenschaft des Polysaccharids während des Verfahrens nicht zerstört wird.

2. Das eingekapselte biologisch aktive Material, das nach 1. erhältlich ist.

3. Die Verwendung des nach 1. eingekapselten biologisch aktiven Materials zur Herstellung von Substanzen.

Die Erfindung wird im folgenden detailliert beschrieben bzw. in den Ansprüchen definiert.

Unter biologisch aktivem Material sind beispielsweise Zellen und Enzyme zu verstehen. Als Stoffe für die Gelkapseln können grundsätzlich alle für das einzukapselnde Material physiologisch akzeptablen Polysaccharide eingesetzt werden, deren Gelphase bei höheren Temperaturen als die der Solphase liegt. Der Sol-/Gel-Übergang sollte bei einer Temperatur liegen, die das einzuschließende Material in seiner Aktivität nicht wesentlich beeinträchtigt. Geeignet sind beispielsweise hydrophob substituierte Polysaccharide, wie Alkyl- und Hydroxyalkylcellulosen oder Hydroxyalkylstärken. Bevorzugt sind Celluloseether wie Methylcellulose, Hydroxybutylmethylcellulose, Hydroxypropylmethylcellulose, Hydroxyethylmethylcellulose und Ethylhydroxyethylcellulose. Besonders bevorzugt werden Hydroxypropylcellulose und Hydroxybutylmethylcellulose.

Das erfindungsgemäße Gelkapselmaterial wird in Konzentrationen von 0,05 bis 10 Gew.-%, bevorzugt 0,1 bis 3 Gew.-%, in einer wäßrigen Lösung gelöst. Die Übergangstemperaturen vom Sol zum Gel liegen in Wasser im Bereich von 10 bis 70°C, bevorzugt bei 20 bis 50°C. Durch Veränderung der Ionenstärke, d.h. durch Zugabe von Salzen, läßt sich der Übergang zu niedrigeren Temperaturen verschieben. Die Temperaturabnahme ist proportional zu der Ionenstärke mit stoffabhängigen Proportionalitätskonstanten im Bereich von

$$5 - 50 \ \frac{grd \cdot L}{Mol}$$

(siehe Abbildung für den Sol-Gel-Übergang am Beispiel der Hydroxypropylcellulose). Für diesen Zweck eignen sich beispielsweise Salze, die den wäßrigen Kulturmedien zugesetzt werden, insbesondere Phosphate, Sulfate und Chloride.

Das einzukapselnde, biologisch aktive Material wird bei einer physiologisch geeigneten Temperatur und bei geeignetem pH-Wert, in der erfindungsgemäß hergestellten wäßrigen Lösung des Kapselmaterials suspendiert. Die einzelnen Bedingungen sind weitgehend von dem biologischen Material abhängig und sind dem Fachmann bekannt bzw. können in einfachen Vorversuchen festgestellt werden. Beispielsweise sind größenordnungsmäßig ein Temperaturbereich von 20 bis 50°C und ein pH-Bereich von 3 bis 9 geeignet. Die wäßrige Lösung enthält vorteilhaft Salze und gegebenenfalls Nährstoffe, die später zur Kultivierung des eingeschlossenen Materials notwendig sind.

Man kann das biologisch aktive Material sowohl in einem Polysaccharid-Block einschließen, beispielsweise zur vorübergehenden Stabilisierung, oder in Kugeln immobilisieren, was für eine spätere Inkubation vorteilhaft ist. Zur Ausbildung von Kugeln wird die Suspension durch eine Kanüle gedrückt. Die austretenden Tropfen mit einer Größe von etwa 100 bis 2000 µm fallen in eine wäßrige Lösung mit einer für das biologisch aktive Material geeigneten Zusammensetzung. Liegt die Temperatur dieser Lösung über der Sol-/Gel-Übergangstemperatur, findet die Gelbildung sofort statt, so daß einzelne Gelkugeln in der Lösung vorliegen. Die Kapseln sammeln sich in dem Medium als separate Phase an, die durch Filtration abgetrennt werden kann.

Je nach Temperaturempfindlichkeit oder Anwendung kann das eingeschlossene biologisch aktive Material direkt zur Inkubation eingesetzt werden oder, falls z.B. die Temperatur, bei der sich das verwendete Polysaccharid im Gelzustand befindet, für das biologische Material zu hoch ist und es dadurch in seiner Aktivität beeinträchtigt wird, kann in einer nächsten Verfahrensstufe eine semipermeable Membran auf die Oberfläche der Polysaccharid-Kapseln aufgebracht werden.

Dies geschieht beispielsweise in wäßriger Lösung durch Aufbringen von Ladungen und anschließender Bildung von Polyelektrolyt-Komplexmembranen um das Polysaccharid, das sich sowohl im Gel- wie auch im Sol-Zustand befinden kann. Hierzu werden die Gelkugeln,bzw. das Sol unter Rühren,in die Lösung eines geladenen Amphiphaten überführt, dessen hydrophobe Gruppen um die hydrophoben Bereiche der substituierten Polysaccharide aggregieren. An diese wird damit eine Ladung geheftet, so daß ein entgegengesetzt geladener Polyelektrolyt elektrostatisch gebunden werden kann. Je nach Ladung werden dann Polybasen oder Polysäuren aufkomplexiert. Die eigentliche Membranbildung erfolgt nach dem Waschen der Kapseln in wäßriger Lösung durch Komplexieren mit einer wiederum entgegengesetzt geladenen Polybase oder Polysäure. Danach oder gleichzeitig kann dann durch Abkühlen der Temperatur des Mediums, in dem sich die Kapseln befinden, das Gel wieder in den Solzustand überführt werden.

Geeignete Amphiphaten müssen zellverträglich sein. Bevorzugt verwendet man positiv geladenes Cetylammoniumbromid oder negativ geladene Phosphatidsäure. Geeignete Polysäuren sind beispielsweise Carrageen, Carboxy-methylcellulose, Alginate, Polyacrylsäure, wobei die zwei erstgenannten Verbindungen bevorzugt sind. Als geeignete Polybasen sind beispielhaft Chitosan, Polyethylenimine oder Polylysin zu nennen mit Bevorzugung der letzteren Verbindung.

Eine alternative Methode zum Aufbringen von Oberflächenladungen besteht darin, bei der Herstellung der Polysaccharid-Kapsel dem Material ein negativ geladenes Polymeres, z.B. Carboxymethylcellulose, beizumischen, wodurch man eine negative Oberflächenladung erhält.

Wird die Polysaccharid-Kapsel nach Ausbildung der dauerhaften komplexen Membranhülle aufgelöst, so muß das Medium, in dem sich die Kapseln befinden, abgekühlt werden und zwar auf eine Temperatur, die unter der Sol-/Gel-Übergangstemperatur liegt. Dabei kann gegebenenfalls eine Volumenänderung beim Sol-/Gel-Übergang zur Änderung der Membranpermeabilität genutzt werden, da durch Quellung des Polysaccharids die Membranpermeabilität erhöht wird.

Eine weitere Möglichkeit die Polysaccharid-Kapseln mit einer zweiten Membran zu umgeben ist deren Bildung aus nicht wäßrigen Lösungen. Die Polysaccharid-Kapseln werden beispielsweise durch Eintropfen des Sols, in dem das biologisch aktive Material suspendiert ist, in ein organisches Lösungsmittel oder Lösungsmittelgemisch gebildet, in dem die Kapseln nicht löslich sind. Durch starkes Rühren erreicht man eine Dispersion von Polysaccharid-Kugeln in dem gewählten Lösungsmittel, wobei das Polysaccharid in Abhängigkeit von der Temperatur sowohl im Sol- wie auch im Gel-Zustand vorliegen kann. Die Wahl des Lösungsmittels ist abhängig von der Art des biologischen Materials. Eine der Voraussetzungen dabei ist, daß die biologische Aktivität durch das Lösungsmittel nicht dauerhaft beeinträchtigt wird. Dies kann in Vorversuchen jedoch leicht ermittelt werden. Geeignete Lösungsmittel sind höhermolekulare, wenig polare Flüssigkeiten z.B. Phthalsäurealkylester, Diisoamylether, Dodecan, Decalin oder Decanol.

Als Polymer für die Ausbildung der äußeren Membran werden ebenfalls Verbindungen eingesetzt, die die biologische Aktivität des Materials nicht nachhaltig vermindern, beispielsweise übliche Cellulosederivate wie Celluloseacetat, Cellulosebutyrat, Ethylcellulose oder Derivate der Polyacrylsäure oder Polymethacrylsäure. Sie sind unlöslich im Polysaccharid-Sol aber partiell löslich in den genannten organischen Lösungsmittel. Die Bildung der Membran um das Polysaccharid-Sol oder Gel erfolgt durch Phasenseparation. Das membranbildende Polymer wird in einem organischen Lösungsmittel zu der beschriebenen Dispersion Lösungsmittel/Polysaccharid gegeben. An dieses organische Lösungsmittel ist ebenfalls die Anforderung zu stellen, daß es für das biologisch aktive Material physiologisch verträglich sein muß und das Polysaccharid in ihm unlöslich ist. Man verwendet zweckmäßig das Lösungsmittel, das ohnehin in der Dispersion schon enthalten ist und in dem das Polymer nur partiell gelöst ist. Das Polymer bildet durch Ausfällung an der Phasengrenzfläche um das Polysaccharid sowohl im Sol-Zustand wie auch im Gel-Zustand eine Membran. Verwendet man für die Zugabe zur Dispersion ein Lösungsmittel, in dem das Polymer vollkommen löslich ist, so kann die Fällung und Ausbildung der Membran schon durch Beimischen der Dispersion oder erst durch Zusatz eines physiologisch verträglichen Fällungsmittels, beispielsweise Wasser oder entsprechende organische Medien, die die Löslichkeit des Polymers herabsetzen, bewirkt werden.

In den folgenden Beispielen wird die Erfindung in Einzelheiten beschrieben. Prozentangaben beziehen sich, wenn nicht anders angegeben, auf das Gewicht.

Beispiel 1

Hydroxypropylcellulose (Klucel M. Mg 800.000, Hercules Inc.) wird 1 %ig bei 37°C in Dulbecco's Phosphatpuffer (Flow Laboratories) gelöst. Der Puffer hat die folgende Zusammensetzung:
$CaCl_2 \cdot H_2O$ 135,5 mg/l
KCl 200,0 mg/l
$KH_2PO_4$ 200,0 mg/l
$MgCl_2 \cdot 6H_2O$ 100,0 mg/l
NaCl 8000,0 mg/l
$Na_2HPO_4$ 1150,0 mg/l
pH 7
Hybridoma-Zellen werden bei 37°C durch Rühren in dieser Lösung suspendiert.

Die Suspension wird aus einer Einwegspritze durch eine Kanüle (0,4 mm Durchmesser) gedrückt. Die austretenden Tropfen (2 mm) fallen in die Pufferlösung mit obengenannter Zusammensetzung, die auf 42°C erwärmt wurde. Die Gelbildung findet sofort statt, so daß einzelne Gelkugeln in der warmen Lösung vorliegen.

Beispiel 2

Die gemäß Beispiel 1 erhaltenen Gelkugeln werden in eine 0,1 %ige wäßrige Cetylammoniumbromidlösung gegeben, in der sie 15 Minuten verbleiben. Nach Waschen mit Dulbecco's Pufferlösung werden sie 10 Minuten in eine 0,2 %ige ι-Carrageenlösung (Fa. Sigma) eingebracht. Nach erneutem Waschen werden die Kugeln in eine 1 %ige Chitosanlösung (Fa. Sigma) überführt, in der sie auf 37°C abgekühlt werden. Wenn die Kugeln nach 15 Minuten ihre ursprüngliche Ausdehnung von 2 mm wieder erreicht haben, werden sie der Chitosanlösung entnommen und nach dem Waschen zur Inkubation des eingeschlossenen Materials in Dulbeccos Puffer mit foetalem Kälberserum überführt. In den Mikrokapseln befinden sich durchschnittlich 50 - 150 Hybridoma-Zellen, deren Wachstum in Roller-Fläschchen über 30 Tage verfolgt wird. Bei einer Zelldichte von etwa $1 \times 10^7$/ml wird eine Syntheseleistung von 500 µg monoklonaler Antikörper/ml ermittelt.

Beispiel 3

Man verfährt gemäß Beispiel 1 mischt der Hydroxypropyl cellulose jedoch Carboxymethylcellulose im Verhältnis 1 : 1 zu. Die Kugeln werden in eine 0,2 %ige Chitosanlösung überführt. Nach Waschen mit Puffer werden die Kugeln zur weiteren Stabilisierung in eine 1 %ige Carboxymethylcellulose-Lösung gebracht. Der weitere Verfahrensablauf erfolgt wie schon in Beispiel 2 beschrieben.

Beispiel 4

In eine Vorlage von 20 ml Dibutylphthalat mit 1 % Dimethylaminoethylmethacrylat-Copolymer (Eudragit, E. Röhm/Darmstadt) werden unter Rühren 0,5 ml einer wäßrigen Lösung von 1 % Hydroxypropylcellulose (Klucel M) und 0,8 % $Na_2SO_4$, in der außerdem Hybridoma-Zellen suspendiert sind, durch eine Kanüle, wie in Beispiel 1 beschrieben, eingetropft.

Die Dispersion wird auf 42°C erwärmt, wobei sich der Durchmesser der mit Copolymer umhüllten Gelkugeln auf ca. 1/3 verringert (ca. 800 µm). Das organische Medium wird abdekantiert und die Gelkugeln bei 42°C mit wäßrigem Puffermedium (Phosphatpuffer pH 7,4) gewaschen und in Dulbecco-Nährmedium mit einer Temperatur von 37°C überführt und gemäß Beispiel 2 mit dem gleichen Ergebnis inkubiert.

Beispiel 5

10 ml einer wäßrigen Lösung von 0,8 % Hydroxybutylmethylcellulose (Methocel HB, Dow Chem. Corp.) und 0,8 % $Na_2SO_4$ werden bei 25°C mit 10 mg Pepsin der spezifischen Aktivität von 63 U/mg (Serva Feinbiochemica GmbH) versetzt. Die spezifische Aktivität wird nach Determann et al. (Hoppe-Seyler's Z. Physiol.Chem. 350, 380 (1969), im Standardhämoglobintest bestimmt.

Die Lösung wird in 50 ml einer 0,8 %igen wäßrigen $Na_2SO_4$ Lösung bei 46°C gegossen. Die gebildeten Gelklumpen mit dem darin stabilisierten Enzym werden nach 24 h durch Dekantieren abgetrennt und in 10 ml physiologischer Kochsalzlösung bei 25°C resuspendiert. Die spezifische Aktivität des dabei aus dem Sol freigesetzten Pepsin beträgt 77 U/mg.

**Patentansprüche für die Vertragsstaaten: BE, CH, DE, FR, GB, IT, LI, LU, NL, SE**

1. Verfahren zum Einkapseln von biologisch aktivem Material, dadurch gekennzeichnet, daß zur Ausbildung der Kapseln ein Polysaccharid mit umgekehrtem thermoreversiblen Temperaturverhalten eingesetzt wird, wobei diese Eigenschaft des Polysaccharids während des Verfahrens nicht zerstört wird.

2. Verfahren nach Anspruch 1, dadurch gekennzeichnet, daß ein hydrophob substituiertes Polysaccharid eingesetzt wird.

3. Verfahren nach Anspruch 2, dadurch gekennzeichnet, daß ein Cellulosether als Polysaccharid eingesetzt wird.

4. Verfahren nach Anspruch 3, dadurch gekennzeichnet, daß Hydroxypropylcellulose oder Hydroxybutylmethylcellulose als Celluloseether eingesetzt wird.

5. Verfahren nach einem oder mehreren der Ansprüche 1 bis 4, dadurch gekennzeichnet, daß um das Polysaccharid im Gel- oder Sol-Zustand eine semipermeable Membran ausgebildet wird.

6. Verfahren nach Anspruch 5, dadurch gekennzeichnet, daß vor oder nach Ausbildung der semipermeablen Membran der Sol-/Gel-Übergang des Polysaccharids stattfindet.

7. Verfahren nach Anspruch 6, dadurch gekennzeichnet, daß der Sol-/Gel-Übergang bei 10 bis 70°C stattfindet.

8. Verfahren nach Anspruch 7, dadurch gekennzeichnet, daß der Sol-/Gel-Übergang bei 20 bis 50°C stattfindet.

9. Verfahren nach Anspruch 5 dadurch gekennzeichnet, daß zur Ausbildung der semipermeablen Membran in wäßriger Lösung

a) mit Hilfe eines Amphiphaten Ladungen auf das Polysaccharid im Gel- oder Sol-Zustand aufgebracht werden und

b) mit Hilfe von Polysäuren oder Polybasen Polyelektrolytkomplexmembranen gebildet werden.

10. Verfahren nach Anspruch 5, dadurch gekennzeichnet, daß ein Polymer, das die biologische Aktivität des eingeschlossenen Materials nicht nachhaltig vermindert, aus organischer Lösung an der Phasengrenzfläche zum Polysaccharid-Sol oder -Gel ausgefällt wird und eine Membran um das Polysaccharid bildet.

11. Eingekapseltes biologisch aktives Material in Gegenwart eines Polysaccharids mit umgekehrtem thermoreversiblen Temperaturverhalten erhältlich nach einem Verfahren eines oder mehrerer der Ansprüche 1–10.

12. Verwendung des eingekapselten biologisch aktiven Materials nach Anspruch 11, zur Herstellung von Substanzen.

**Patentansprüche für die Vertragsstaaten: AT, ES, GR**

1. Verfahren zum Einkapseln von biologisch aktivem Material, dadurch gekennzeichnet, daß zur Ausbildung der Kapseln ein Polysaccharid mit umgekehrtem thermoreversiblen Temperaturverhalten eingesetzt wird, wobei diese Eigenschaft des Polysaccharids während des Verfahrens nicht zerstört wird.

2. Verfahren nach Anspruch 1, dadurch gekennzeichnet, daß ein hydrophob substituiertes Polysaccharid eingesetzt wird.

3. Verfahren nach Anspruch 2, dadurch gekennzeichnet, daß ein Cellulosether als Polysaccharid eingesetzt wird.

4. Verfahren nach Anspruch 3, dadurch gekennzeichnet, daß Hydroxypropylcellulose oder Hydroxybutylmethylcellulose als Celluloseether eingesetzt wird.

5. Verfahren nach einem oder mehreren der Ansprüche 1 bis 4, dadurch gekennzeichnet, daß um das Polysaccharid im Gel- oder Sol-Zustand eine semipermeable Membran ausgebildet wird.

6. Verfahren nach Anspruch 5, dadurch gekennzeichnet, daß vor oder nach Ausbildung der semipermeablen Membran der Sol-/Gel-Übergang des Polysaccharids stattfindet.

7. Verfahren nach Anspruch 6, dadurch gekennzeichnet, daß der Sol-/Gel-Übergang bei 10 bis 70°C stattfindet.

8. Verfahren nach Anspruch 7, dadurch gekennzeichnet, daß der Sol-/Gel-Übergang bei 20 bis 50°C stattfindet.

9. Verfahren nach Anspruch 5, dadurch gekennzeichnet, daß zur Ausbildung der semipermeablen Membran in wäßriger Lösung

a) mit Hilfe eines Amphiphaten Ladungen auf das Polysaccharid im Gel- oder Sol-Zustand aufgebracht werden und

b) mit Hilfe von Polysäuren oder Polybasen Polyelektrolytkomplexmembranen gebildet werden.

10. Verfahren nach Anspruch 5, dadurch gekennzeichnet, daß ein Polymer, das die biologische Aktivität des eingeschlossenen Materials nicht nachhaltig vermindert, aus organischer Lösung an der Phasengrenzfläche zum Polysaccharid-Sol oder -Gel ausgefällt wird und eine Membran um das Polysaccharid bildet.

**Claims for the Contracting States: BE, CH, DE, FR, GB, IT, LI, LU, NL, SE**

1. A process for encapsulating biologically active material, which comprises using a polysaccharide with an inverse thermoreversible temperature behavior for forming the capsules, this property of the polysaccharide not being destroyed during the process.

2. The process as claimed in claim 1, wherein a hydrophobically substituted polysaccharide is used.

3. The process as claimed in claim 2, wherein a cellulose ether is used as the polysaccharide.

4. The process as claimed in claim 3, wherein hydroxypropylcellulose or hydroxybutylmethylcellulose is used as the cellulose ether.

5. The process as claimed in one or more of claims 1 to 4, wherein a semipermeable membrane is formed around the polysaccharide in the gel state or sol state.

6. The process as claimed in claim 5, wherein the sol/gel transition of the polysaccharide takes place before or after the formation of the semipermeable membrane.

7. The process as claimed in claim 6, wherein the sol/gel transition takes place at 10 to 70°C.

8. The process as claimed in claim 7, wherein the sol/gel transition takes place at 20 to 50°C.

9. The process as claimed in claim 5, wherein, for forming the semipermeable membrane in aqueous solution,

    a) charges are applied to the polysaccharide in the gel or sol state by means of an amphipathic substance and

    b) polyelectrolyte complex membranes are formed by means of polyacids or polybases.

10. The process as claimed in claim 5, wherein a polymer, which does not cause a lasting reduction in the biological activity of the enclosed material, is precipitated from organic solution at the phase boundary with the polysaccharide sol or gel and forms a membrane around the polysaccharide.

11. An encapsulated biologically active material, obtainable in the presence of a polysaccharide with an inverse thermoreversible temperature behavior by the process as claimed in one or more of claims 1 to 10.

12. The use of the encapsulated biologically active material as claimed in claim 11 for the preparation of substances.

**Claims for the Contracting States: AT, ES, GR**

1. A process for encapsulating biologically active material, which comprises using a polysaccharide with an inverse thermoreversible temperature behavior for forming the capsules, this property of the polysaccharide not being destroyed during the process.

2. The process as claimed in claim 1, wherein a hydrophobically substituted polysaccharide is used.

3. The process as claimed in claim 2, wherein a cellulose ether is used as the polysaccharide.

4. The process as claimed in claim 3, wherein hydroxypropylcellulose or hydroxybutylmethylcellulose is used as the cellulose ether.

5. The process as claimed in one or more of claims 1 to 4, wherein a semipermeable membrane is formed around the polysaccharide in the gel state or sol state.

6. The process as claimed in claim 5, wherein the sol/gel transition of the polysaccharide takes place before or after the formation of the semipermeable membrane.

7. The process as claimed in claim 6, wherein the sol/gel transition takes place at 10 to 70°C.

8. The process as claimed in claim 7, wherein the sol/gel transition takes place at 20 to 50°C.

9. The process as claimed in claim 5, wherein, for forming the semipermeable membrane in aqueous solution,

    a) charges are applied to the polysaccharide in the gel or sol state by means of an amphipathic substance and

    b) polyelectrolyte complex membranes are formed by means of polyacids or polybases.

10. The process as claimed in claim 5, wherein a polymer, which does not cause a lasting reduction in the biological activity of the enclosed material, is precipitated from organic solution at the phase boundary with the polysaccharide sol or gel and forms a membrane around the polysaccharide.

**Revendications pour les Etats contractants: BE, CH, DE, FR, GB, IT, LI, LU, NL, SE**

1. Procédé d'encapsulation d'un matériau biologiquement actif, caractérisé en ce que, pour la formation des capsules, on met en œuvre un polysaccharide à comportement thermoréversible dans les deux sens, cette propriété n'étant pas affectée en cours de procédé.

2. Procédé selon la revendication 1, caractérisé en ce que l'on met en œuvre un polysaccharide à substitution hydrophobe.

3. Procédé selon la revendication 2, caractérisé en ce que l'on met en œuvre, comme polysaccharide, un éther de cellulose.

4. Procédé selon la revendication 3, caractérisé en ce que l'on met en œuvre, comme éther de cellulose, l'hydroxypropylcellulose ou l'hydroxybutylméthylcellulose.

5. Procédé selon l'une ou plusieurs des revendications 1 à 4, caractérisé en ce que l'on forme autour du polysaccharide, à l'état de gel ou de sol, une membrane semiperméable.

6. Procédé selon la revendication 5, caractérisé en ce que la transition sol/gel du polysaccharide a lieu avant ou après formation de la membrane semiperméable.

7. Procédé selon la revendication 6, caractérisé en ce que la transition sol/gel du polysaccharide a lieu entre 10 et 70°C.

8. Procédé selon la revendication 7, caractérisé en ce que la transition sol/gel a lieu entre 20 et 50°C.

9. Procédé selon la revendication 5, caractérisé en ce que, pour la formation de la membrane semiperméable en solution aqueuse,

    a) on dépose, à l'aide d'un amphiphate, des charges sur le polysaccharide à l'état gel ou sol, et

    b) on forme des membranes polyélectrolytiques complexes à l'aide de polyacides ou de polybases.

10. Procédé selon la revendication 5, caractérisé en ce que l'on précipite à partir d'une solution organique, à l'interphase du sol ou du gel de polysaccharide, un polymère qui ne réduit pas durablement l'activité biologique du matériau incorporé et qui forme une membrane autour du polysaccharide.

11. Matériau biologiquement actif encapsulé en présence d'un polysaccharide à comportement thermoréversible dans les deux sens, qui peut être obtenu par un procédé selon l'une ou plusieurs des revendications 1 à 10.

12. Emploi du matériau biologiquement actif encapsulé de la revendication 11, pour la fabrication de substances.

**Revendications pour les Etats contractants: AT, ES, GR**

1. Procédé d'encapsulation d'un matériau biologiquement actif, caractérisé en ce que, pour la formation des capsules, on met en œuvre un polysaccharide à comportement thermoréversible dans les deux sens, cette propriété n'étant pas affectée en cours de procédé.

2. Procédé selon la revendication 1, caractérisé en ce que l'on met en œuvre un polysaccharide à substitution hydrophobe.

3. Procédé selon la revendication 2, caractérisé en ce que l'on met en œuvre, comme polysaccharide, un éther de cellulose.

4. Procédé selon la revendication 3, caractérisé en ce que l'on met en œuvre, comme éther de cellulose, l'hydroxypropylcellulose ou l'hydroxybutylméthylcellulose.

5. Procédé selon l'une ou plusieurs des revendications 1 à 4, caractérisé en ce que l'on forme autour du polysaccharide, à l'état de gel ou de sol, une membrane semiperméable.

6. Procédé selon la revendication 5, caractérisé en ce que la transition sol/gel du polysaccharide a lieu avant ou après formation de la membrane semiperméable.

7. Procédé selon la revendication 6, caractérisé en ce que la transition sol/gel du polysaccharide a lieu entre 10 et 70°C.

8. Procédé selon la revendication 7, caractérisé en ce que la transition sol/gel a lieu entre 20 et 50°C.

9. Procédé selon la revendication 5, caractérisé en ce que, pour la formation de la membrane semiperméable en solution aqueuse,

a) on dépose, à l'aide d'un amphiphate, des charges sur le polysaccharide à l'état gel ou sol, et

b) on forme des membranes polyélectrolytiques complexes à l'aide de polyacides ou de polybases.

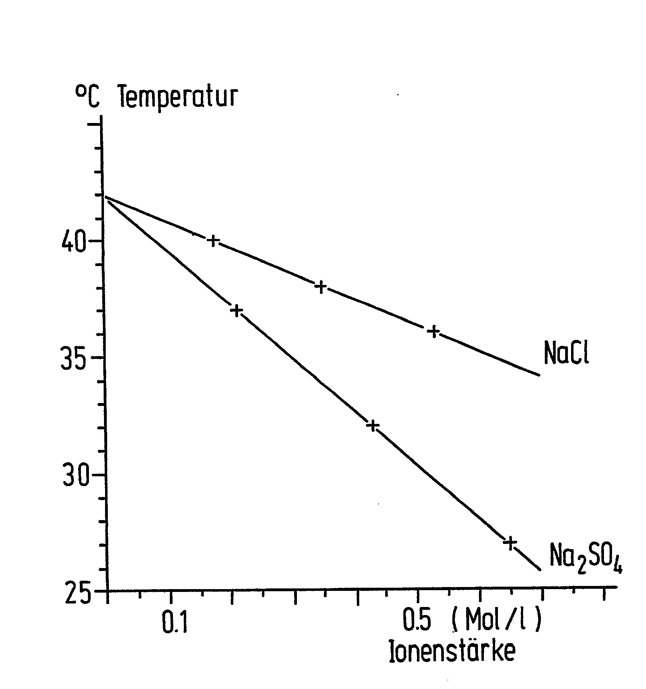

10. Procédé selon la revendication 5, caractérisé en ce que l'on précipite à partir d'une solution organique, à l'interphase du sol ou du gel de polysaccharide, un polymère qui ne réduit pas durablement l'activité biologique du matériau incorporé et qui forme une membrane autour du polysaccharide.